(19) Europäisches Patentamt
European Patent Office
Office européen des brevets

(11) Publication number: 0 124 283
A1

# (12) EUROPEAN PATENT APPLICATION

(21) Application number: 84302210.4

(22) Date of filing: 30.03.84

(51) Int. Cl.³: C 07 C 127/22

(30) Priority: 31.03.83 US 480734

(43) Date of publication of application: 07.11.84
Bulletin 84/45

(84) Designated Contracting States: AT BE CH DE FR GB IT LI LU NL SE

(71) Applicant: UNION CARBIDE CORPORATION, Old Ridgebury Road, Danbury Connecticut 06817 (US)

(72) Inventor: Chan, John Kai-Fai, 9612 Post Mill Place, Raleigh North Carolina 27614 (US)

(74) Representative: Baverstock, Michael George Douglas et al, BOULT, WADE & TENNANT 27 Furnival Street, London, EC4A 1PQ (GB)

## (54) Process for the preparation of benzoyl ureas.

(57) A process for the preparation of benzoyl ureas is provided wherein the condensed intermediate formed by the reaction of a halogen-substituted nitrobenzene with a hydroxy-substituted cyclic or bicyclic compound is contacted with water, forming a solid or semi-solid which is easily isolated. Reduction of the nitro compound to the corresponding amine and subsequent carbamoylation of the amine with an isocyanate are all conducted in the same solvent.

EP 0 124 283 A1

# PROCESS FOR THE PREPARATION OF BENZOYL UREAS

This invention relates in general to a process for the preparation of benzoyl ureas which are useful as the active toxicant in pesticidal compositions. In one aspect, this invention relates to an improved three-step process for the preparation of benzoyl ureas, such as, 1-(3-chloro-4-[4-chloro-1-naphthoxy]-2,5-dimethylphenyl)-3-(2,6-difluoro benzoyl) urea.

Recently a series of new insect growth regulant compounds have been disclosed in the patent literature. For example, U.S. - A - 4,275,077 which issued on June 23, 1981 to H.M. Becker et al and is assigned to Celemerk GmbH & Co., K.G. discloses and claims certain insecticidal aryl urea derivatives. Also of interest is a novel class of bicyclooxyphenyl benzoyl ureas which were recently disclosed in European Patent application number 0050321 which was published on April 28, 1982 in European Patent Bulletin No. 1982/17. These compounds are substituted aryl ureas and are prepared by a general three-step reaction involving (1) the condensation of an appropriate halogen-substituted nitrobenzene with a hydroxy-substituted polycyclic compound; (2) the reduction of the resulting condensed product with hydrogen in the presence of a catalyst to form the corresponding amine; and (3) the carbamoylation of the amine with an appropriate isocyanate compound to form the desired urea compound.

The condensation reaction is usually conducted at high temperatures (i.e. greater than 100°C) in the presence of dimethylformamide (DMF) or dimethyl sulfoxide (DMSO) as the reaction solvent. Unfortunately, the use of either of these solvents complicates the isolation of the desired nitro compound at the end of the condensation step since it is very difficult to remove either DMF or DMSO completely from the reaction mixture. Thus the recovery of an acceptable nitro derivative usually requires very cumbersome operations such as low-pressure stripping, solvent exchange, column chromatography, and recrystallization. The use of any of these recovery procedures is uneconomical and almost always leads to a low yield of the desired end product. Additionally, in many cases, the product obtained is not of the desired degree of purity. Consequently, there existed a need for a simpler and more effective process for the preparation of the precursor desired urea compounds.

Accordingly, one or more of the following objects can be achieved by the practice of this invention. It is an object of this invention to provide a process for the preparation of benzoyl ureas. Another object of this invention is to provide an improved three-step process for the synthesis of benzoyl ureas. A further object is to provide an improved process for the preparation of compounds such as 1-(3-chloro-4-[4-chloro-1-naphthoxy]-2,5-dimethylphenyl)-3-(2,6-difluoro benzoyl)urea. A still further object is to provide an improved process for the preparation of benzoyl ureas which are useful as the active toxicant in

pesticidal compositions. These and other objects will readily become apparent to those skilled in the art in the light of the teachings herein set forth.

In its broad aspect this invention relates to a process for the preparation of benzoyl ureas which are useful as the active toxicant in pesticidal compositions. The process involves contacting the condensed intermediate formed by the reaction of the halogen-substituted nitrobenzene with a hydroxy-substituted cyclic or bicyclic compound with water whereby a solid or semi-solid is formed which can be conveniently isolated. The solid is dissolved in a suitable solvent in which reduction of the nitro compound to the amine and subsequent carbamoylation of the amine derivative with the appropriate isocyanate compound can be effected. This process provides the desired urea in relatively high yields and of a high degree of purity.

As indicated above, the process of this invention is directed to a multi-step process for the preparation of benzoyl urea compounds wherein relatively high yields of a pure product are obtained. The process comprises the steps of:

a) contacting in a condensation solvent a halogen-substituted nitrobenzene compound of the general formula:

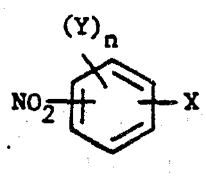

(I)

with a hydroxy-substituted cyclic or polycyclic compound of the respective formulae:

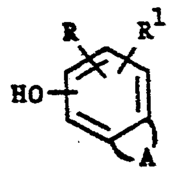

(II)

or

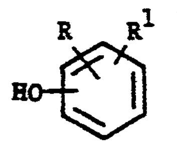

(IIa)

under conditions wherein condensed nitrophenyl compounds are formed and which have the respective formulae:

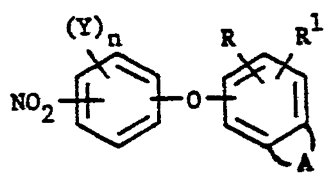

(III)

or

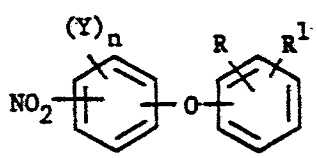

(IIIa)

b) contacting said condensed nitrophenyl compound with an amount of water sufficient to form a solid or semi-solid, of said compound;

c) dissolving said solid in a second solvent, and reducing said nitrophenyl compound to the corresponding amine;

d) contacing said amine in said same second solvent with an isocyanate compound of the formula:

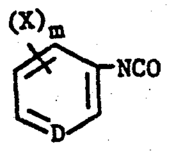

(IV)

and recovering benzoyl urea compounds of the respective formulae:

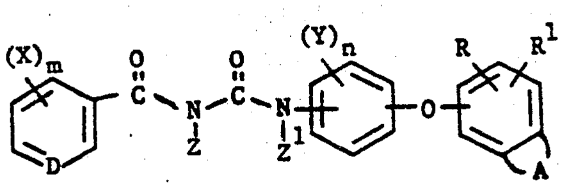

(V)

or

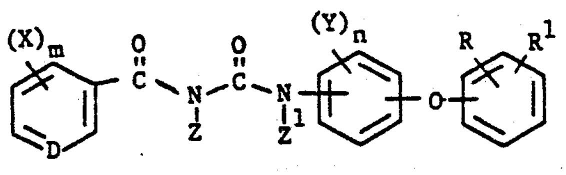

(Va)

wherein:

D is nitrogen or carbon;

X individually represents halogen, nitro, cyano, or alkyl, polyhaloalkyl, alkoxy or polyhaloalkoxy of from 1 to 3 carbon atoms;

m has a value of from 1 to 4;

Z and $Z^1$ individually represent hydrogen or alkyl of from 1 to 8 carbon atoms, or alkyl of from 1 to 8 carbon atoms which is substituted with at least one of halogen, hydroxyl or alkoxy;

Y individually represents halogen, or alkyl, polyhaloalkyl, alkoxy or polyhaloalkoxy of from 1 to 3 carbon atoms;

n has a value of from 0 to 4;

R and $R^1$ individually represent halogen, nitro, cyano, amino, formamido, formamidino, phenylsulfenyl, phenylsulfinyl, phenylsulfonyl, phenylsulfamido, wherein the phenyl ring optionally may be substituted with one or more halogen, nitro, or alkyl, polyhaloalkyl, alkoxy or polyhaloalkoxy of from 1 to 3 carbon atoms, or R and $R^1$ individually are alkyl, alkoxy, polyhaloalkyl, polyhaloalkoxy, alkenyloxy, polyhaloalkenyloxy, alkynyloxy, polyhaloalkynyloxy, alkylsulfenyl, polyhaloalkylsulfenyl, alksulfinyl, polyhaloalkylsulfinyl, alkylsulfonyl, polyhaloalkylsulfonyl, mono- or di-alkylsulfamido, mono- or di-alkylamino, alkylcarbonylamino, alkoxycarbonylamino, or mono- or di-alkylaminocarbonyloxy of up to 6 carbon atoms; and

A, together with the carbon atoms to which it is attached, forms a five or six-membered carboxyclic ring or a six-membered saturated or unsaturated heterocyclic ring which can contain in any combination carbonyl or one or two oxygen or sulfur, and up to two $R^2$ and $R^3$ substituents attached to unsaturated ring carbon atoms, wherein $R^2$ and $R^3$ individually can be halogen, nitro, cyano, or alkyl, polyhaloalkyl, alkoxy, polyhaloalkoxy, alkylthio, arylthio, alkylsulfinyl,

arylsulfinyl, alkyl-sulfonyl, arylsulfonyl, alkylsulfamido, arylsulfamido, alkoxycarbonylamino, or alkylcarbonylamino of from 1 to 6 carbon atoms, or the ring can contain up to two $R^4$ and $R^5$ substituents attached to saturated ring carbon atoms, wherein $R^4$ and $R^5$ individually can be alkyl or polyhaloalkyl of from 1 to 6 carbon atoms.

The process of the present invention enables a much easier recovery of the nitroether derivative and leads to improved yields of the final urea product. As indicated above, the condensation reaction is conducted in a condensation solvent, such as dimethylformamide, followed by introducing water into the reaction mixture to give the solid product. The water can be added hot, or the mixture heated after the water has been added. It has been found that an advantage of using water to precipitate the product is that inorganic impurities are soluble in the water and hence easily separated from the desired product. The solid product is then dissolved in a second solvent and reduced with hydrogen in the presence of a suitable catalyst to form the amine derivative. After azeotropic drying, the derivative is finally carbamoylated in-situ with the appropriate benzoyl isocyanate to give the desired product in high yields.

By the term "condensation solvent" as employed throughout the specification and appended claims is meant a solvent which is polar and contains a carbonyl group. Although dimethylformamide is chosen for use as the condensation solvent, other solvents such as acetone, methyl ethyl ketone, methyl isopropyl ketone, dimethyl sulfoxide or sulfolane,

may also be used successfully. The preferred amount of reaction solvent to be used for the condensation is 3 to 20 times with respect to the total amount of reactants used in the reaction. The most preferred amount is 3 to 8 times by weight. When less than the preferred amount of solvent is used, the condensation may not proceed well. Of course, greater volume of solvent could be used although it is less economical.

The condensation reaction is best conducted at a temperature range of 50 to 200°C; the preferred temperature range is 75 to 150°C. At these temperature ranges, a reaction time of 3 to 72 hours is required to complete the condensation reaction. The reaction time is very much dependent upon the reactiveness of the halogen-substituted nitrobenzene used as starting material.

At the end of the condensation, the hot reaction mixture can be poured into 2-5 parts of water with respect to the total volume of the reaction mixture to form a solid. Conversely water can be introduced into the reaction mixture. The aqueous layer is then removed by decantation or filtration. The solid is then dissolved in a second solvent for further reactions leading to the final compound. An alternate procedure is first to remove or partially remove the condensation solvent under reduced pressure and then adding hot water to the residue to dissolve the inorganic matter and to crystallize the nitroether intermediate which can then be recovered by decantation or filtration for further reactions.

As previously mentioned the solid product obtained from the condensation step is dissolved in

a second solvent in which both the reduction and carbamoylation reaction can be conducted. The second solvent can be any inert organic solvent which does not adversely effect the reactants and in which the reactants are soluble and the final product is insoluble. Illustrative solvents include but are not limited to, compounds such as toluene, xylene, ethyl acetate, methylene chloride, cyclohexanone and acetone.

The reaction temperature for the hydrogenation step may range from 10 to 200°C under 20 to 1000 psi pressure; the preferred range is 25 to 75°C under 50 to 250 psi. At these temperature and pressure ranges, a reaction time of 1 to 24 hours is required to complete the reduction step. Catalysts that are useful for the reduction step are Raney nickel, platinum, palladium, and other common hydrogenation catalysts. Useful catalyst supports are carbon and barium sulfate.

Toluene is a preferred solvent for dissolving the crude nitroether intermediate for the hydrogenation step. It also has an advantage that the reduced product, the amino-ether derivative, is very soluble whereas the final compound is insoluble. Consequently, at the end of the hydrogenation step, after the water formed has been azeotropically removed, the reaction mixture can be carbamoylated in-situ to form the desired product, which can then be recovered as a fine-quality product via a simple filtration step.

The final carbamoylation reaction can be conducted at a temperature range of 10 to 100°C. The preferred temperature range is 20 to 80°C. At these temperature ranges, a reaction time of from 1

to 6 hours is required to complete the carbamoylation. Under the preferred conditions, longer times do not affect the product yields; too short reaction times may render poor yields of the desired product.

As previously indicated, the process of the present invention is useful for the preparation of those benzoyl ureas wherein a halogen-substituted nitrobenzene is condensed with an hydroxy-substituted cyclic or polycyclic compound, the condensed product reduced to the corresponding amine, and the amine subsequently reacted with a benzoyl isocyanate.

Preferred benzoyl ureas which can be prepared by the process of the present invention are those represented by formulae V or Va above. Certain of the benzoyl urea represented by formula V are those disclosed in the aforementioned European Patent application Number 005031 which corresponds to copending United States patent application Serial No. 305,951, filed September 28, 1981 and assigned to the same assignee as the present invention. The benzoyl ureas represented by formula Va are those disclosed in copending United States patent application Serial No. 393,553 filed June 30, 1982 also assigned to the same assignee as the present invention.

Also, preferred ureas are those of the formula:

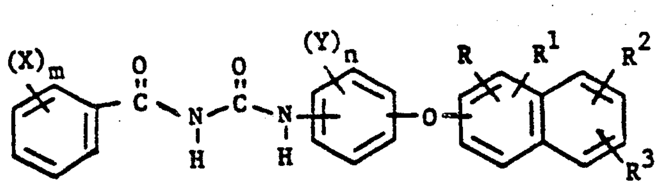

(VI)

or

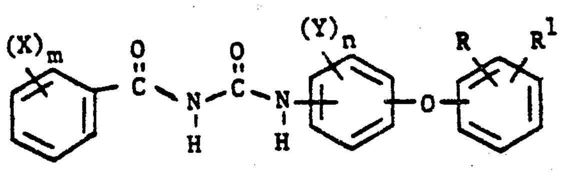

(VII)

wherein X, Y, $R-R^3$, m and n are as defined above.

Particularly preferred ureas which can be prepared by the process of this invention are those of the formula:

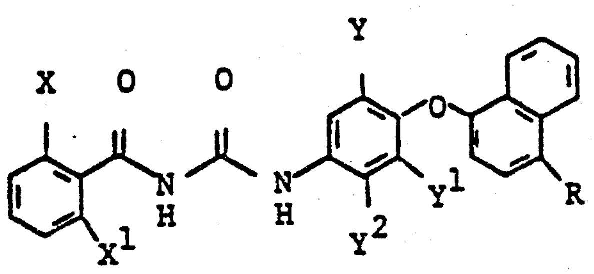

(VIII)

wherein R is chloro, alkoxy or dialkylamino of from 1 to 6 carbon atoms, X is chloro or fluoro, $X^1$ is hydrogen, chloro or fluoro, Y is hydrogen, chloro, bromo, methyl or methoxy, $Y^1$ and $Y^2$ independently are hydrogen, chloro, bromo or methyl.

0124283

Also particularly preferred are the benzoyl ureas of the formula:

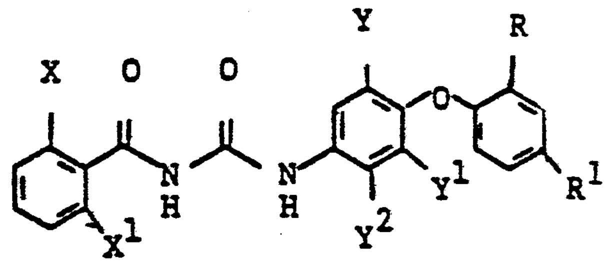

(IX)

wherein R, $R^1$, Y and $Y^2$ are alkyl of from 1 to 4 carbon atoms, $Y^1$ is halogen, X is halogen and $X^1$ is hydrogen or halogen.

Illustrative benzoyl ureas which can be prepared by the process of this invention, include, among others,

1-[4-(4-chloro-1-naphthoxy)-3,5-dichlorophenyl]-3-(2,6-difluorobenzoyl)urea,

1-[4-(4-chloro-1-naphthoxy)-3-methylphenyl]-3-(2-chlorobenzoyl)urea,

1-[3-chloro-4-(4-chloro-1-naphthoxy)-2,5-dimethylphenyl]-3-(2-chlorobenzoyl)urea,

1-[4-(2,4-dimethylphenoxy)-3,6-dimethyl-5-chlorophenyl]-3-(2,6-difluorobenzoyl)urea,

1-[4-(2,4-dimethylphenoxy)-3-6-dimethyl-5-chlorophenyl]-3-(2-chlorobenzoyl)urea,

1-(3,5-dichloro-4-[4-nitro-1-naphthoxy]phenyl-3-(2-chloro-benzoyl)urea,

1-(3,5-dichloro-4-[4-nitro-1-naphthoxy]phenyl)-3-(2,6-dichloro-benxoyl)urea,

1-(3,5-dichloro-4-[4-nitro-1-naphthoxy]phenyl)-3-(2,6-difluorobenzoyl)urea,

1-(3,5-dichloro--4-[4-nitro-1-naphthoxy]phenyl)-3-(2-chloro-6-fluorobenzoyl)urea,

1-(3,5-dichloro-4-[4-cyano-1-naphthoxy]phenyl)-3-(2-chlorobenzoyl)urea,

1-(3,5-dichloro-4-[4-cyano-1-naphthoxy]phenyl)-3-(2,6-dichlorobenzoyl)urea,

1-(3,5-dichloro-4-[4-cyano-1-naphthoxy]phenyl)-3-(2,6-difluorobenzoyl)urea,

1-(3,5-dichloro-4-[4-cyano-1-naphthoxy]phenyl)-3-(2-chloro-6-fluorobenzoyl)urea,

1-(3,5-dichloro-4-[4-trifluoromethyl-1-naphthoxy] phenyl-3-(2-chlorobenzoyl)urea,

1-(3,5-dichloro-4-[4-trifluoromethyl-1-naphthoxy] phenyl-3-(2,6-dichlorobenzoyl)urea,

1-(3,5-dichloro-4-[4-trifluoromethyl-1-naphthoxy] phenyl-3-(2,6-difluorobenzoyl)urea,

1-(3,5-dichloro-4-[4-trifluoromethyl-1-naphthoxy] phenyl-3-(2-chloro-6-fluorobenzoyl)urea,

1-(4-[1-naphthoxy]-3-trifluoromethylphenyl)-3-(2,6-dimethoxybenzoyl)urea,

1-(3,5-dichloro-4-[4-chloro-2,2-dimethyl-2,3-dihydro-7-benzofuranyloxy]phenyl)-3-(2-chlorobenzoyl)urea,

1-(3,5-dichloro-4-[2,2-dimethyl-2,3-dihydro-4-nitro-7-benzofuranyloxy]phenyl)-3-(2,6 dichlorobenzoyl) urea,

1-(3,5-dichloro-4-[4-cyano-2,2-dimethyl-2,3-dihydro-7-benzofuranyloxy]phenyl)-3-(2,6 difluorobenzoyl) urea,

1-(3,5-dichloro-4-[2,2-dimethyl-2,3-dihydro-4-trifluoromethyl-7-benzofuranyloxy]phenyl-3-(2-chloro-6-fluorobenzoyl)urea,

1-(3,5-dibromo-4-[2,3-dihydro-2,2-dimethyl-7-benzofuranyloxy]-phenyl)-3-(2,6-dimethoxybenzoyl)urea,

1-(3,5-dichloro-4-[5,6,7,8-tetrahydro-4-trifluoromethyl-1-naphthoxy]-phenyl)-3-(2,6-dichlorobenzoyl) urea,

1-(3,5-dichloro-4-[4-chloro-5,6,7,8-tetrahydro-1-naphthoxyl]-phenyl)-3-(2,6-difluorobenzoyl)urea,

1-(3,5-dichloro-4-)4-nitro-4,6,7,8-tetrahydro-1-naphthoxy]-phenyl)-3-(2-chloro-6-fluorobenzoyl) urea,

1-(3,5-dichloro-4-[4-cyano-5,6,7,8-tetrahydro-1-naphthoxy]-3-(2-chlorobenzoyl)urea,

1-(3-chloro-4-[2,4-dichloro-5,6,7,8-tetrahydro-1-naphthoxy]-3-(2,6-dimethoxybenzoyl)urea,

1-(3-bromo-4-[3-bromo-7-benzofuranyloxy]-5-methyl-phenyl)-3-benzoylurea,

1-(3-chloro-4-[2-chloro-4-phenylsulfenyl-6-benzo-furanyloxy]-5-ethylphenyl)-3-(2,6-diisopropyl-benzoyl)urea,

1-(3,5-dimethoxy-4-[3-fluoro-4-benzofuranyloxy]-3-(2-trifluoromethoxybenzoyl)urea,

1-(4-[7-(2,4-dichlorophenylsulfenyl)-3-ispropoxy-5-benzofuranyloxy]phenyl)-3-(2-ethoxybenzoyl)urea,

1-(3-iodo-4-[4-methylsulfenyl-7-benzothienyloxy-[phenyl)-3-(2-chloro-6-fluorobenzoyl)urea,

1-(3,5-difluoro-4-[7-fluoro-4-trifluoromethyl-6-benzothienyloxy]phenyl)-3-(2-difluoromethylbenzoyl) urea,

1-(4-[ 6,7-dichloro-3-nitro-4-benzothienyloxy]-3-heptafluoropropoxyphenyl)-3-(2,6-dibromobenzoyl) urea,

1-(3,5-dichloro-4-[4-benzothienyloxy]phenyl)-1-(2-chloro-6-fluorobenzoyl)urea,

1-(3,5-dichloro-4-[7-chloro-4-benzothienyloxy]phenyl-1-(2,6-difluorobenzoyl)urea,

1-(3-chloro-4-[7-chloro-4-benzothienyloxy]phenyl-1-(2,6-dimethoxybenzoyl)urea,

1-(4-[6-bromo-4-chloro-2,3-difluoro-5-benzothienyl-oxy]-3-isopropoxyphenyl)-3-(2-bromobenzoyl)urea,

1-(4-[5-fluoro-4-benzodioxyalanyloxy]-3-isopropylphenyl)-3-(2,6-bistrifluoromethylbenzoyl)urea,

1-(3,5-dichloro-4-[benzodioxalanyloxy]phenyl)-3-(2,6-difluorobenzoyl)urea,

1-(4-[2,2-bistrifluoromethyl-5-benzodioxalanyloxy]-3,5-dichloro-phenyl)-3-(2-chloro-6-fluorobenzoyl)-urea,

1-[4-(2-methylphenoxy)-3,6-dimethyl-5-chlorophenyl]-3-(2,6-difluorobenzoyl)urea,

1-[4-(2-methylphenoxy)-3,6-dimethyl-5-chlorophenyl]-3-(2,6-dichlorobenzoyl)urea,

1-[4-(2-methylphenoxy)-3,6-dimethyl-5-chlorophenyl]-3-(2-chlorobenzoyl)urea,

1-[4-(2-methylphenoxy)-3,6-dimethyl-5-chlorophenyl]-3-(2-chlorobenzoyl)thiourea,

1-[4-(2-methylphenoxy)-3,6-dimethyl-5-chlorophenyl]-3-(2-methylbenzoyl)thiourea,

1-[4-(2-methylphenoxy)-2,3,5,6-tetramethylphenyl]-3-(2,6-dimethylbenzoyl)urea,

1-[4-(2-methylphenoxy)-2,3,5,6-tetramethylphenyl]-3-(2,6-methylbenzoyl)thiourea,

1-[4-(2-methylphenoxy)-2,3,5,6-tetramethylphenyl]-3-(2,6-difluorobenzoyl)urea,

1-[4-(2-methylphenoxy)-2,3,5,6-tetramethylphenyl]-3-(2,6-dichlorobenzoyl)urea,

1-[4-(2-methylphenoxy)-2,3,5-trimethylphenyl]-3-(2,6-dichlorobenzoyl)urea,

1-[4-(2-methylphenoxy)-3,5-dichlorophenyl]-3-(2,6-difluorobenzoyl)urea,

1-[4-(2-methyl-4-bromophenoxy)-3,6-dimethyl-5-chlorophenyl]-3-(2,6-difluorobenzoyl)urea,

1-[4-(2-methyl-4-bromophenoxy)-3,6-dimethyl-5-chlorophenyl]-3-(2-chlorobenzoyl)urea,

1-[4-(2-methyl-4-bromophenoxy)-3,6-dimethyl-5-chlorophenyl]-3-(2-chlorobenzoyl)thiourea,

1-[4-(2-methyl-4-bromophenoxy)-3,6-dimethyl-5-chlorophenyl]-3-(2-methylbenxoyl)thiourea,

1-[4-(2-methyl-4-bromophenoxy)-2,3,5,6-tetramethylphenyl]-3-(2,6-difluorobenxoyl)urea,

1-[4-(2-methyl-4-bromophenoxy)-2,3,5-tetramethylphenyl]-3-(2,6-difluorobenxoyl)urea,

1-[4-(2-methyl-4-bromophenoxy)-3,5-dimethylphenyl]-3-(2,6-difluorobenzoyl)urea,

1-[4-(2-methyl-4-bromophenoxy)-3,5-dichlorophenyl]-3-(2-chlorobenzoyl)urea,

1-[4-(2-bromo-4-methylphenoxy)-3,6-dimethyl-5-chlorophenyl]-3-(2,6-difluorobenzoyl)urea, and

1-[4-(2-bromo-4-methylphenoxy)-3,6-dimethyl-5-chlorophenyl]-3-(2-chlorobenzoyl)urea.

As previously indicated the compounds which can be prepared by the process of this invention are useful as the active toxicant in pesticidal compositions. The compounds disclosed in U.S. - A - 4,275,077 were indicated to be useful for control of larvae aides aegyptii, caterpillers spodoptere littorclis $L_3$ and bug larvae epilachna varivestis $L_3$. The compounds disclosed in European Patent application No. 0050321 were shown to be particularly useful in control of insects and mites.

The following examples illustrate the best mode presently contemplated for the practice of this invention:

## Example 1

### Preparation of 1-(4-[4-chloro-1-naphthoxy]--3-methylphenyl)-3-(2-chlorobenzoyl)urea

A mixture of 2-bromo-5-nitrotoluene (6.5g, 0.03 mole), 4-chloro-1-naphthol (5.4g, 0.03 mole),

potassium carbonate (4.2g) and 70 ml of dimethylformamide was heated at 140°C for 24 hours. After heating, the hot reaction mixture was poured into 100g of water with stirring. A gummy solid was formed on the bottom of the container and the dark aqueous layer was decanted. The gummy solid was then dissolved in 100 ml of toluene and transferred to a 500-ml Parr hydrogenation bottle together with a small amount of 5% plantinum-on-carbon catalyst. The mixture as hydrogenated under 50 psi hydrogen pressure at 25-30°C until no more hydrogen was absorbed. The catalyst was removed by filtration and the filtrate was dried over magnesium sulfate. The dried toluene solution was finally reacted with 2-chlorobenzoyl isocyante (5g) at ambient temperature over a 1-hour period to give 11g of 1-(4-[4-chloro-1-naphthoxy]-3-methylphenyl-3-(2-chloro benzoyl)urea, with a melting point of 210-214°C. The overall yield was 78.8% based on the amount of 2-bromo-5-nitrotoluene used. The product structure was confirmed by spectral analyses.

Calcd. for $C_{25}H_{18}Cl_2N_2O_3$: C, 64.34%; H, 3.87%; N, 6.02%

Found: C, 64.72%; H, 4.03%; N, 5.82%

Example 2

Preparation of 1-(3-chloro-4-[4-chloro-1-naphthoxy]- -2,5-dimethylphenyl)-3-(2-chlorobenzoyl)urea

The process of Example 1 was repeated using 2,3-dichloro-5-nitro-1,4-xylene (6.6g, 0.03 mole), anhydrous potassium carbonate (4.2g), 4-chloro-1-naphthol (0.03 mole) and 70g of dimethyformamides as starting materials, all other reaction conditions being the same. At the end of

the three-step reaction, a quantity of 5g of compound, was obtained with a melting point of 184-187°C. The product structure was substantiated by spectral analyses and by mixed melting with an authentic sample. The overall yield was 32 percent based on the amount of 2,3-dichloro-5-nitro-1,4-xylene used.

Example 3

Preparation of 1-(4-[4-chloro-1-naphthoxy[-3,5--dichlorophenyl)-3-(2,6-difluorobenzoyl)urea

The process of Example 1 was repeated using a mixture consisting of 3,4,5-trichloronitro-benzene (6.8g, 0.030 mole), 4-chloro-1-naphthol (5.3g, 0.03 mole), anhydrous potassium carbonate (4.3g) and 65g of dimethylformamide as starting materials, all other reaction conditions being the same. 2,6-Difluorobenzoyl isocyanate was used as the isocyanate reactant. At the end of the three-step synthesis, an 8.4g of final product was obtained, with a melting point of 252-255°C. The product was identified as 1-(4-[4-chloro-1-naphthoxy]-3,5-dichlorophenyl)-3-(2,6-difluorobenzoyl)urea. The product structure was substantiated by NMR and by mixed melting point. The overall yield was 76 percent based on the amount of 3,4,5-trichloronitro benzene used.

In a manner similar to that employed in the above examples, a wide variety of other urea compounds can be prepared.

CLAIMS:

1. A process for the preparation of a benzoyl urea of the general formula:

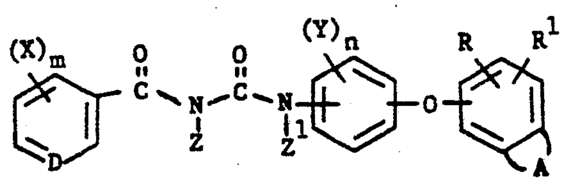

or

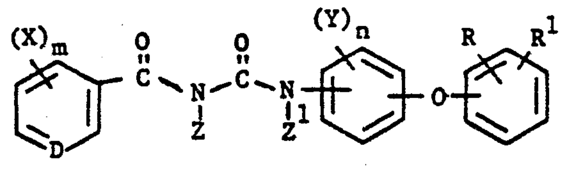

which comprises the steps of:

(a) condensing a halogen-substituted nitrobenzene compound of the formula:

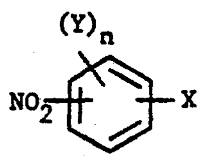

with a hydroxy-substituted cyclic or polycylic compound of the formula:

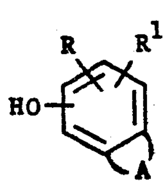

or

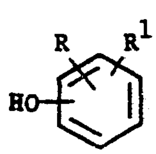

in the presence of a condensation solvent, to form a condensed nitrophenyl compound of the general formula:

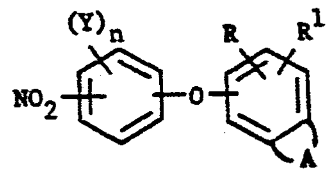

or

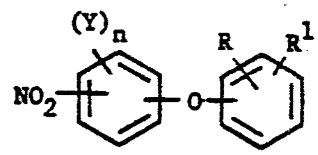

(b) contacting said solvent containing said nitrophenyl compound with water to form a solid,

(c) dissolving said solid in a solvent and reducing said nitrophenyl compound to the corresponding amine of the general formula:

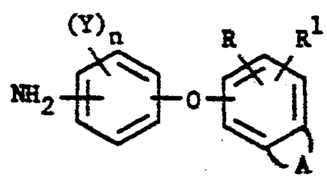

or

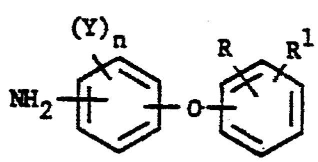

(d) contacting said amine with an isocyanate compound of the formula:

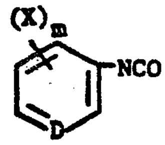

and recovering said benzoyl urea, wherein:
D is nitrogen or carbon;
X individually represents halogen, nitro, cyano or alkyl, polyhaloalkyl, alkoxy or polyhaloalkoxy of from 1 to 3 carbon atoms;
m has a value of from 1 to 4;
Z and $Z^1$ individually represent hydrogen or alkyl of from 1 to 8 carbon atoms, or alkyl of from 1 to 8 carbon atoms which is substituted with at least one of halogen, hydroxyl or alkoxy;
Y individually represents halogen, or alkyl, polyhaloalkyl, alkoxy or polyhaloalkoxy of from 1 to 3 carbon atoms;
n has a value of from 0 to 4;
R and $R^1$ individually represent halogen, nitro, cyano, amino, formamido, formamidino, phenylsulfenyl, phenylsulfinyl, phenylsulfonyl, phenylsulfamido, wherein the phenyl ring optionally may be substituted with one ore more halogen, nitro, or alkyl, polyhaloalkyl, alkoxy or polyhaloalkoxy of from 1 to 3 carbon atoms, or R and $R^1$ individually are alkyl, alkoxy, polyhaloalkyl, polyhaloalkoxy, alkenyloxy, polyhaloalkenyloxy, alkynyloxy, polyhaloalkynyloxy, alkylsulfenyl, polyhaloaklylsulflenyl, alkylsulfinyl, polyhaloalkylsuflinyl, alkylsulfonyl, polyhaloaklylsulfonyl, mono- or di-alkylsulfamido, mono- or di-alkylamino, alkylcarbonylamino, alkoxycarbonylamino or mono- or di-alkylaminocarbonyloxy of up to 6 carbon atoms; and A, together with the carbon atoms to which it is attached, forms a five or six-membered carbocylic ring or a five or six-membered saturated or unsaturated heterocyclic ring which can contain in any combination carbonyl or one or two oxygen or sulfur, and up to two $R^2$ and $R^3$ substituents attached to unsaturated ring carbon atoms, wherein $R^2$ and $R^3$ individually can be halogen, nitro, cyano, or alkyl, polyhaloalkyl, alkoxy, polyhaloalkoxy, alkylthio, arylthio, alkylsulfinyl, arylsulfinyl, alkylsulfonyl, arylsulfonyl alkylsulfamido, arylsulfamido, alkoxycarbonylamino, or alkylcarbonylamino of from 1 to 6 carbon atoms, or the ring can contain up to two $R^4$ and $R^5$ substituents attached to saturated ring carbon atoms, wherein $R^4$ and $R^5$ individually can be alkyl or polyhaloalkyl of from 1 to 6 carbon atoms.

2. The process of claim 1 wherein said benzoyl urea has the general formula:

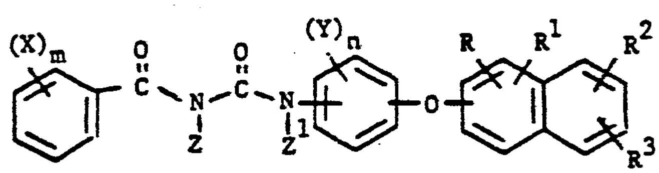

wherein X, Y, Z, $Z^1$, R-$R^3$, n and m are as defined in claim 1.

3. The process of claim 1 wherein said benzoyl urea has the general formula:

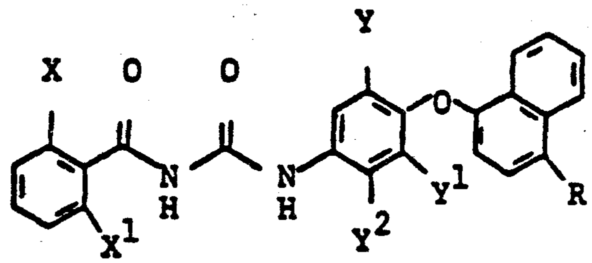

wherein R is halo, alkoxy, or dialkylamine of from 1 to 6 carbon atoms, X is chloro or fluoro, $X^1$ is hydrogen, chloro, or fluoro, Y is hydrogen, chloro, bromo, methyl or methoxy and $Y^1$ and $Y^2$ independently are hydrogen, chloro, bromo or methyl.

4. The process of claim 1 wherein said benzoyl urea has the general formula:

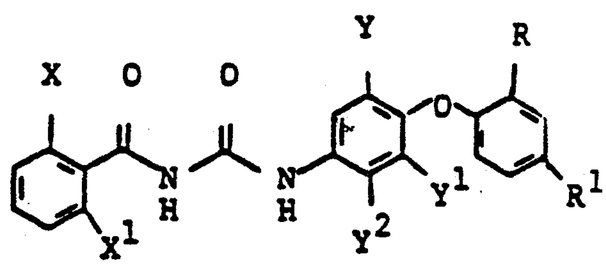

wherein R, $R^1$, Y and $Y^2$ are alkyl of from 1 to 4 carbon atoms, $Y^1$ is halogen, X is halogen and $X^1$ is hydrogen or halogen.

5. The process of claim 1 wherein said benzoyl urea is 1-[4-(4-chloro-1-naphthoxy)-3,5-dichlorophenyl]-3-(2,6-difluorobenzoyl)urea.

6. The process of claim 1 wherein said benzoyl urea is 1-[4-(4-chloro-1-naphthoxy)-3-methyl-phenyl]-3-(2-chlorobenzoyl)urea.

7. The process of claim 1 wherein said benzoyl urea is 1-[3-chloro-4-(4-chloro-1-naphthoxy)-2,5-dimethyl-phenyl]-3-(2-chlorobenzoyl)urea.

8. The process of claim 1 wherein said benzoyl urea is 1-[4-(2,4-dimethylphenoxy)-3,6-dimethyl-5-chlorophenyl]-3-(2,6-difluorobenzoyl)urea.

9. The process of claim 1 wherein said benzoyl urea is 1-[4-(2,4-dimethylphenoxy)-3-6-di-metyl-5-chloro-phenyl]-3-(2-chlorobenzoyl)urea.

10. A process as claimed in any one of the preceding claims wherein the condensation solvent is a ketone.

11. The process of claim 10 wherein the condensation solvent is dimethylformamide.

12. A process as claimed in any one of the preceding claims wherein the condensation solvent is employed in an amount of at least three times the total weight of the reactants.

13. A process as claimed in any one of the preceding claims wherein the water is step (b) is added to the reaction mixture.

14. A process as claimed in any one of claims 1 to 12 wherein the reaction mixture is added to water.

15. A process as claimed in any one of the preceding claims wherein the condensation solvent is at least partially removed from the mixture and water then added to the mixture.

16. A process as claimed in any one of the preceding claims wherein the volume of water employed in step (b) is from 2 to 5 times the total volume of the reaction.

17. A process as claimed in any one of the preceding claims wherein steps (c) and (d) are conducted in toluene as said second solvent.

0124283

European Patent Office

# EUROPEAN SEARCH REPORT

Application number

EP 84 30 2210

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| X | GB-A-1 542 768 (CIBA-GEIGY)<br>* Example 1 and the following *<br>----- | 1-17 | C 07 C 127/22 |
| | | | TECHNICAL FIELDS SEARCHED (Int. Cl. 3) |
| | | | C 07 C 127/00 |

The present search report has been drawn up for all claims

| Place of search | Date of completion of the search | Examiner |
|---|---|---|
| THE HAGUE | 09-07-1984 | GAUTIER R.H.A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82